# FASCICULE DE BREVET EUROPEEN

(11) **EP 0 559 753 B1**
(45) Date de publication et mention de la délivrance du brevet: **12.04.1995**
(21) Numéro de dépôt: 92900704.5
(22) Date de dépôt: 25.11.1991
(51) Int. Cl.: A61M 5/32

(54) **SERINGUE AVEC DISPOSITIF PROTECTEUR D'AIGUILLE**
SPRITZE MIT NADELSCHUTZVORRICHTUNG
SYRINGE WITH A NEEDLE PROTECTOR DEVICE

(30) Priorité: 26.11.1990 FR 9014767
(43) Date de publication de la demande: 15.09.1993
(73) Titulaire: DENTOPTIC, F-60250 Hondainville (FR)
(72) Inventeur: BERTHIER, Michel 33, rue de Gazois, F-62580 Vimy (FR)
(74) Mandataire: Robert, Jean-Pierre
(86) Numéro de dépôt international: FR9100934
(87) Numéro de publication internationale: WO9209319

(56) Documents cités:
- EP-A- 0 250 104
- EP-A- 0 276 160
- WO-A-90/13325
- US-A- 4 894 055
- US-A- 4 911 693
- US-A- 4 932 940

## Description

La présente invention concerne un dispositif protecteur pour aiguille de seringue d'injection.

Actuellement, les seringues sont livrées avec un protecteur permettant leur conservation stérile avant utilisation et leur recouvrement après utilisation pour protéger le manipulateur de la seringue usagée contre toute contamination pouvant résulter d'une piqûre intempestive.

Ces mesures ne sont pas de nature à éliminer tout risque de contamination après usage car il faut, pour l'utilisateur, procéder au recapuchonnage de l'aiguille, opérations au cours de laquelle le risque de se piquer est grand. En outre, les seringues ainsi recapuchonnées restent réutilisables, ce qu'il faut éviter au maximum. C'est pourquoi, dans certains cas, l'aiguille dans un protecteur est rapportée sur la seringue et, après usage, est séparée de la seringue et est cassée pour en interdire la réutilisation. Il reste pour l'opérateur, le risque de se piquer au cours de la manipulation finale.

On rappellera par ailleurs que certaines seringues actuelles comportent un étui cylindrique dont l'une des extrémités est fermée soit par une cloison dans laquelle est implantée l'aiguille d'injection, soit par l'embout porteur de l'aiguille rapportée, et ouvert à son autre extrémité pour recevoir le flacon de produit à injecter, dont la paroi perforable est tourné vers la partie de l'aiguille qui fait saillie dans l'étui, l'étui étant ensuite refermé par un bouchon qui comporte un piston pour agir sur une paroi mobile du flacon opposée à sa paroi perforable.

L'invention est un perfectionnement d'une part aux seringues classiques et, d'autre part, à ce type de seringues composites pour assurer la protection de l'aiguille avant utilisation, sa mise hors d'atteinte après utilisation et l'impossibilité de sa réutilisation.

Il est connu d'isoler l'aiguille au moyen d'une gaine tubulaire coulissante sur le corps de la seringue, qui est placée autour de l'aiguille à partir d'une position dans laquelle elle la découvre. On citera par exemple les seringues du type de celles décrites dans les documents US-A- 4 911 693 et EP-A- 0 250 104.

Le dispositif selon le premier document est adapté à des seringues dont l'aiguille est longue car il est exclusivement porté par cette aiguille. Il ne peut donc pas convenir pour des seringues à cartouches telles qu'elles sont utilisées notamment en art dentaire.

Le dispositif décrit dans le second document ne possède pas de garantie sérieuse quant à l'impossibilité de réutiliser la seringue, car les organes de verrouillages sont accessibles au manipulateur.

L'invention entend remédier à ces inconvénients et à cet effet elle propose un dispositif protecteur pour l'aiguille d'une seringue d'injection, comportant de manière connue un corps cylindrique fermé à l'une de ses extrémités par une paroi transversale dans laquelle l'aiguille est implantée et équipé d'un protecteur d'aiguille susceptible de glisser le long du corps entre deux positions éloignées axialement l'une de l'autre, formant gaine mobile de protection de l'extrémité de l'aiguille, des éléments de guidage et d'indexation étant prévus entre le corps et le protecteur pour n'autoriser qu'un seul dégainage et rengainage successifs de l'aiguille. Selon l'une des caractéristiques principales de l'invention, les éléments d'indexation comportent un organe solidaire du protecteur, situé dans une zone de celui-ci inaccessible de l'extérieur et faisant saillie par rapport à sa surface intérieure pour porter élastiquement sur une surface de guidage de l'autre élément, cette surface étant pourvue dans sa partie extrème voisine de l'aiguille d'un évidement de réception irréversible de l'organe verrouillant le protecteur dans sa position de gainage complet de l'aiguille.

Dans le cas d'une aiguille à cartouche, cette disposition permet de mettre en place le flacon de produit à injecter dans le porte-aiguille sans avoir découvert l'aiguille, puis de découvrir l'aiguille sur une longueur suffisante correspondant à sa profondeur de pénétration dans les tissus et enfin, l'injection effectuée, de rengainer l'aiguille afin de protéger le manipulateur et de rendre la seringue inutilisable.

Dans un mode de réalisation de l'invention, le protecteur possède une première partie tubulaire de grand diamètre portant l'organe de verrouillage, prolongée par une partie de petit diamètre formant gaine mobile de l'extrémité de l'aiguille.

Cet organe peut prendre toute forme de réalisation utile: ce peut être par exemple un anneau fendu radialement et élastiquement déformable qui, logé à la manière d'un circlips dans une gorge du protecteur, peut coulisser le long du corps jusqu'à être conduit dans une gorge circulaire de ce dernier dans laquelle il se contracte, le protecteur étant ainsi maintenu dans sa position de dégainage de l'aiguille, et de laquelle il peut être extrait en repoussant le protecteur en direction de l'aiguille pour se loger de manière alors irréversible dans une autre gorge du corps qui constitue l'évidement mentionné ci-dessus, maintenant ainsi le protecteur dans sa position de condamnation de l'aiguille.

Dans une autre forme de réalisation, l'organe de verrouillage est constitué par l'extrémité libre d'une languette élastique en projection radiale à l'intérieur de la partie de grand diamètre du protecteur, la surface de guidage étant constituée par une rainure ménagée dans le corps et comportant deux branches longitudinales parallèles réunies par une partie circonférentielle à leur extrémité la plus éloignée de l'aiguille, l'une des branches longitudinales de cette rainure comportant à son autre extrémité l'évidement de réception de l'extrémité libre de la languette élastique.

Dans une variante de cette forme de réalisation, la branche dépourvue d'évidement comporte une pluralité de crans, chacun d'eux constituant une butée coopérant avec la languette élastique pour effacer celle-ci dans le sens du dégainage de l'aiguille et pour créer un blocage du coulissement du protecteur en direction de l'aiguille. Cette variante permet de pouvoir règler la longueur d'aiguille à découvrir en fonction de la profondeur de sa pénétration dans les tissus lors de l'injection.

Dans une variante de réalisation, la surface de guidage est ménagée sur une glissière rapportée sur la surface extérieure de la paroi du corps cylindrique de la seringue. Cette possibilité autorise l'adaptation du dispositif selon l'invention à toutes les seringues du marché, sans avoir à recourir à une fabrication spéciale de ces corps de seringue.

D'autres caratéristiques et avantages de l'invention ressortiront de la description des exemples de réalisation donnés ci-après.

Il sera fait référence aux dessins annexés, dans lesquels:
- la figure 1 est une vue schématique partielle en coupe axiale d'un mode de réalisation du dispositif selon l'invention,
- la figure 2 illustre une seconde réalisation du dispositif selon l'invention,
- la figure 3 illustre une variante de réalisation de la figure 2.

Aux figures, on a représenté un injecteur 1 comportant de manière connue, deux parties 2 et 3, la partie 2 étant destinée à recevoir le flacon 4 de produit à injecter, la partie 3 étant destinée à refermer la partie 2 et à fournir le piston 5 de propulsion du liquide qui agira sur une paroi mobile du flacon 4.

La partie 2 forme le support de l'aiguille d'injection 6 qui perfore la paroi perforable du flacon et qui s'étend à l'extérieur du support 2. Cette portion externe de l'aiguille est à protéger contre l'atmosphère extérieure (avant usage) et à rendre inaccessible (après usage) pour protéger le manipulateur et rendre son réemploi impossible.

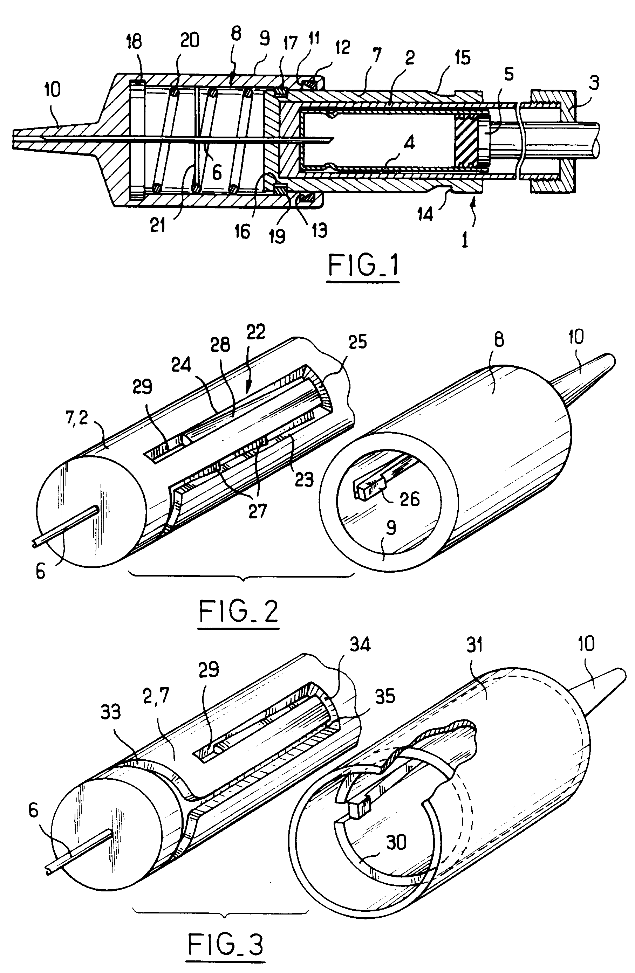

Pour ce faire, conformément à l'invention, le support 2 de l'aiguille est équipé d'une glissière 7 cylindrique qui, sur la figure 1, est rapportée sur la paroi du support 2 alors que sur la figure 2 elle constitue la paroi elle-même.

Cette glissière 7 comporte des moyens pour guider et arrêter en translation un protecteur 8 d'aiguille en deux parties (30, 31), comme représenté en figure 3, qui est de forme cylindrique avec une partie de grand diamètre 9 qui coopère en coulissement avec la glissière 7 et une partie de petit diamètre 10, qui prolonge longitudinalement la partie de grand diamètre 9 pour recouvrir l'aiguille 6.

La partie de grand diamètre 9 du protecteur 8 possède sur une longueur L, un diamètre intérieur égal au diamètre extérieur de la surface cylindrique de la glissière 7, formant une portée de guidage en coulissement de l'une sur l'autre. Cette portée est creusée d'une rainure 11, inaccessible de l'extérieur du protecteur, dans laquelle est logée une bague élastique fendue 12 dont le diamètre libre est inférieur au diamètre extérieur de la glissière 7. Cette bague est chamfreinée sur sa face arrière 13. La glissière possède, à son extrémité opposée à l'aiguille, une rainure 14 dans laquelle peut partiellement se contracter la bague 12 lorsqu'elle est à son niveau. La bague forme ainsi clavette d'indexation axiale du protecteur sur la glissière. La rainure 14 possède également une paroi conique 15 qui forme une rampe de glissement pour le chamfrein 13, afin que la bague 12 puisse être extraite de la rainure 14 lors d'un mouvement du protecteur en direction de l'aiguille.

La glissière 7 comporte une seconde rainure 16 à son extrémité proche de l'aiguille. Cette rainure, dont les bords sont radiaux, peut également accueillir une portion de la bague 12 lorsque celle-ci se contracte à son niveau. La bague 12 est alors logée partiellement de manière irréversible dans cette rainure 16 et la liaison axiale du protecteur à la glissière est définitive.

On notera que la rainure 16 est, sur la figure 1, occupée par une bague secondaire 17 qui est aussi élastique mais qui, à l'inverse de la bague 12, tend à se dilater diamétralement contre la paroi interne du protecteur 8. Cette paroi interne est creusée d'une rainure secondaire 18 qui se trouvera en face de la bague 17 lorsque la bague 12 sera en face de la rainure 14. A ce moment, la bague 17 s'escamotera totalement dans cette rainure 18 et sera entraînée par le protecteur 8 dans son mouvement de rengainage de l'aiguille. Le rôle de cette bague 17 est d'une part, de permettre une introduction facile du protecteur 8 sur la glissière 7, au moment du montage du dispositif, en empèchant la bague 12 de se contracter dans la rainure 16, et d'autre part, de constituer une butée d'arrèt, pour empêcher la dissociation du protecteur et de la glissière, en coopérant avec un petit épaulement interne 19 que présente intérieurement la paroi du protecteur 8.

La glissière 7 peut comporter plus d'une rainure telle que 16 de manière à permettre plusieurs longueurs de dégainage de l'aiguille. Dans ce cas la rainure 18 sera placée de manière à accueillir la bague 17 lorsque la bague 12 aura atteint la première rainure de la glissière 7.

On notera enfin la présence d'un ressort 20 entre la glissière 7 et la protecteur 8, logé dans ce dernier, et tendant à repousser le protecteur en direction de l'aiguille. Un disque 21 au moins est disposé entre l'aiguille et le protecteur, à l'ntérieur de celui-ci pour former élément de maintien de l'aiguille contre son flambement lors de son introduction dans les tissus du patient. Ce disque possède une découpe sur son bord pour le passage du ressort.

On comprend, au vu de cette figure, que le protecteur 8 peut être facilement repoussé vers la seringue pour découvrir une longueur plus ou moins importante de seringue avant l'injection et, après usage, être ramené ou, sous l'effet du ressort, revenir automatiquement à sa position initiale pour recouvrir de manière définitive l'aiguille. Les moyens de verrouillage étant inaccessibles, il est impossibles de réutiliser facilement la seringue.

A la variante de la figure 2, les moyens de guidage et d'indexation entre le protecteur et la glissière, qui est ici le corps 2 lui-même de la seringue, sont constitués, sur la glissière, par une rainure en "U" 22 dont les deux branches 23 et 24 s'étendent le long de deux génératrices distinctes de la glissière et sont réunies par une rainure circonférentielle 25 les reliant transversalement, et sur le protecteur, par une languette élastique 26 faisant saillie, à l'intérieur du protecteur, dans la branche 23 de la rainure 22. Dans cette branche, le coulissement dans le sens du dégainage de l'aiguille est possible sans entrave. De manière avantageuse, surtout lorsqu'un ressort tel que 20 est mis en oeuvre, le fond de la branche 23 est équipé de crans 27 avec lesquels la languette 26 coopère à la manière d'un cliquet, interdisant le retour du protecteur en direction de l'aiguille et s'effaçant au passage des crans dans l'autre sens du mouvement. Dans l'autre branche, le fond 28 est en légère pente ascendante en direction de l'aiguille, pour ensuite se terminer par un évidement 29 duquel le cliquet ne peut être extrait. Le mouvement du protecteur est alors définitivement condamné. Pour parvenir à cette condamnation, il convient de repousser le protecteur 8 vers la partie circonférentielle 25 de la rainure 22 et de le faire tourner pour placer la languette 26 en regard de la branche 24, autorisant ainsi le rengainage de l'aiguille assisté par le ressort.

A la figure 3 on a représenté la réalisation comme revendiquée dans laquelle le protecteur est en deux pièces: l'une intérieure 30 qui est en un matériau résistant, par exemple un polycarbonate, comportant le cliquet de verrouillage, l'autre extérieure 31, dans une matière semblable ou différente, solidarisée à la partie intérieure par collage, soudage,...qui constitue un manchon portant la partie de petit diamètre et enfermant la pièce intérieure, rendant ainsi inaccessible le cliquet de verrouillage en le recouvrant. Le corps de la seringue ou la glissière 2, 7 comporte une rainure 32 en U comme dans le cas précédent, mais dont une des branches prend racine dans une gorge circulaire 33 qui constitue le logement du cliquet dans la position du protecteur avant usage, l'extrémité de l'autre branche étant pourvue de l'évidement 29 d'où le cliquet ne peut être extrait. Pour éviter de replacer le protecteur dans sa première position, la partie circonférentielle 34 qui relie les deux branches de la rainure peut définir, avec l'extrémité de la première branche, un cran 35 de non-retour du protecteur en direction de sa première position

L'invention n'est pas limitée à la description qui vient d'en être donnée. Elle couvre également des variantes de réalisation non-représentées telles que celles mettant en oeuvre d'autres moyens de guidage et d'indexation du protecteur sur la glissière (inserts ou cliquets métalliques...). De même, la disposition des cliquet et glissière peut, par rapport à celle décrite, être inversée.

## Revendications

1. Dispositif protecteur pour l'aiguille d'une seringue d'injection, comportant un support cylindrique (2) d'aiguille fermé à l'une de ses extrémités par une paroi transversale dans laquelle l'aiguille (6) est implantée, équipé d'un protecteur (8) d'aiguille susceptible de glisser le long d'une surface extérieure de guidage du support (2, 7) entre deux positions éloignées axialement l'une de l'autre, formant gaine mobile de protection de l'extrémité de l'aiguille (6), des éléments (11, 12, 14, 16, 22, 26) de guidage et d'indexation étant prévus entre le support (2, 7) et le protecteur pour n'autoriser qu'un seul dégainage et rengainage successifs de l'aiguille, ces éléments d'indexation comportant un organe (12, 26) solidaire du protecteur et faisant saillie par rapport à sa surface intérieure pour porter élastiquement sur une surface (22) de guidage de l'autre élément, cette surface étant pourvue dans sa partie extrême voisine de l'aiguille (6) d'un évidement (16, 19) de réception irréversible de l'organe verrouillant le protecteur (8) dans sa position de gainage complet de l'aiguille, caractérisé en ce que l'organe de verrouillage est constitué par l'extrémité libre d'une languette (26) élastique en projection radiale à l'intérieur du protecteur, montée sur une première partie (30) intérieure du protecteur recouverte par un manchon extérieur (31), la surface de guidage étant constituée par une rainure (22) ménagée sur le support (2, 7) et comportant deux branches (23, 24) longitudinales parallèles réunies par une partie circonférencielle (25) à leur extrémité la plus éloignée de l'aiguille, l'une (24) des branches longitudinales de cette rainure comportant à son autre extrémité l'évidement (29) de réception de l'extrémité libre de la languette élastique.

2. Dispositif selon la revendication 1, caractérisé en ce que le manchon extérieur (31) possède une première partie tubulaire de grand diamètre (9) dans laquelle est logée la partie intérieure (30) portant l'organe de verrouillage, prolongée par une partie de petit diamètre (10) formant gaine mobile de l'extrémité de l'aiguille (6).

3. Dispositif selon la revendication 1 ou la revendication 2, caractérisé en ce que la branche (23) dépourvue d'évidement comporte une pluralité de crans (27), chacun d'eux constituant une butée coopérant avec la languette élastique (26) pour effacer celle-ci dans le sens de dégainage de l'aiguille et pour créer un blocage du coulissement du protecteur en direction de l'aiguille.

4. Dispositif selon l'une quelconque des revendications précédentes, caractérisé en ce que la surface de guidage est ménagée sur une glissière (7) rapportée sur la surface extérieure du support (2) d'aiguille.

5. Dispositif selon l'une quelconque des revendications 2 à 4, caractérisé en ce qu'il comprend, attelé entre le protecteur (8) et le support (2, 7) d'aiguille, dans la partie (9) de grand diamètre de ce protecteur, un organe (20) de rappel élastique du protecteur dans sa position de gainage complet de l'aiguille.

6. Dispositif selon l'une quelconque des revendications 2 à 5, caractérisé en ce que la partie de grand diamètre du protecteur comprend au moins un élément de maintien (21) de l'aiguille (6) au centre du protecteur.

## Claims

1. A protection device for the needle of an injection syringe, including a cylindrical support (2) for a needle (6) closed at one of its end by a transverse wall in which the needle (6) is implanted, fitted with a needle protector (8) suitable for sliding along an outside guiding surface of the support support (2, 7) between two positions, that are mutually separated in the axial direction, forming a moving protective sheath for the end of the needle (6), guiding and indexing elements (11, 12, 14, 16, 22, 26) being provided between the support (2, 7) and the protector to allow the needle to be uncovered and subsequently covered again by the sheath once only, the indexing elements including a member (12, 26) secured to the protector and projecting from its inside surface to bear resiliently against a guide surface (22) of said guiding elements, said surface being provided in its end portion adjacent to the needle (6) with a recess (16, 19) for irreversibly receiving the locking member of the protector (8) in its position where the needle is fully covered, characterized in that the locking member is constituted by the free end of a resilient tongue (26) projecting radially inside the protector, mounted on a first inner portion (30) of the protector covered by an outer sleeve (31), the guide surface being constituted by a groove (22) formed in the support (2, 7) and having two parallel longitudinal branches (23, 24) interconnected by a circumferential portion (25) at their ends furthest from the needle, one of the longitudinal branches (24) of said groove including the recess (29) at its opposite end for receiving the free end of the resilient tongue.

2. A device according to claim 1, characterized in that the outer sleeve (31) has a tubular first portion of large diameter in which is housed the inner portion (30) carrying the locking member and extended by a portion of small diameter (10) forming the moving sheath for the end of the needle (6).

3. A device according to claim 1 or to claim 2, characterized in that the branch (23) that does not include the recess includes a plurality of ratchets (27) each constituting an abutment co-operating with the resilient tongue (26) to cause it to be retracted in the needle-uncovering direction while preventing the protector from sliding in the needle-covering direction.

4. A device according to any preceding claim, characterized in that the guide surface is formed on a slideway (7) applied to the outside surface of the cylindrical support (2) of the needle.

5. A device according to any one of claims 2 to 4, characterized in that is includes a resilient return member (20) coupled between the protector (8) and the needle support (2, 7) in the large diameter portion (9) of said protector, said member urging the protector into its position where it covers the needle in full.

6. A device according to any one of claims 2 to 5, characterized in that the large diameter portion of the protector includes at least one element (21) for holding the needle (6) in the center of the protector.

## Patentansprüche

1. Vorrichtung zum Schutz der Nadel einer Injektionsspritze, mit einem zylindrischen Nadel-Trägerteil (2), das an einem seiner Enden durch eine transversale Wand, in welche die Nadel (6) eingesetzt ist, geschlossen und mit einem Nadelschutz (8) versehen ist, der entlang einer äußeren Führungsfläche des Trägerteils (2, 7) zwischen zwei axial voneinander entfernten Positionen verschiebbar ist und eine bewegliche Schutzhülle für das Ende der Nadel (6) bildet, wobei Elemente (11, 12, 14, 16, 22, 26) zur Führung und Indexierung zwischen dem Trägerteil (2, 7) und dem Nadelschutz vorgesehen sind, die dafür sorgen, daß die Nadel nur ein einziges Mal enthüllt und anschließend wieder verhüllt werden kann, wobei diese Indexierungselemente ein Organ (12, 26) umfassen, das mit dem Nadelschutz fest verbunden ist und gegenüber der Innenfläche desselben vorspringt, um sich elastisch an eine Führungsfläche (22) des anderen Elements anzulegen, wobei diese Fläche in ihrem der Nadel (6) benachbarten Endbereich eine Ausnehmung (16, 19) zur irreversiblen Aufnahme des den Nadelschutz (8) in seiner die Nadel vollständig umhüllenden Position verriegelnden Organs hat, dadurch gekennzeichnet, daß das Verriegelungsorgan durch das freie Ende einer an der Innenseite des Nadelschutzes radial vorstehenden elastischen Zunge (26) gebildet ist, die an einem ersten inneren Abschnitt (30) des von einer äußeren Hülse (31) umschlossenen Nadelschutzes montiert ist, wobei die Führungsfläche von einer in dem Trägerteil (2, 7) ausgebildeten Nut (22) mit zwei an ihrem von der Nadel am weitesten entfernten Ende durch einen umfangsseitigen Abschnitt (25) vereinten parallelen Längszweigen (23, 24) gebildet ist, deren einer (24) an seinem anderen Ende die Ausnehmung (29) für die Aufnahme des freien Endes der elastischen Zunge aufweist.

2. Vorrichtung nach Anspruch 1, dadurch gekennzeichnet, daß die äußere Hülse (31) einen ersten rohrförmigen Abschnitt (9) mit großem Durchmesser hat, in welchem der das Verriegelungsorgan tragende innere Bereich (30) aufgenommen ist und der durch einen Abschnitt (10) mit kleinem Durchmesser verlängert ist, der eine bewegliche Hülse für das Ende der Nadel (6) bildet.

3. Vorrichtung nach Anspruch 1 oder 2, dadurch gekennzeichnet, daß der ausnehmungsfreie Zweig (23) eine Vielzahl von Rastkerben (27) hat, deren jede einen mit der elastischen Zunge (26) zusammenwirkenden Anschlag bildet, um die Zunge in Enthüllungsrichtung der Nadel beiseite zu drücken und eine Sperre gegen die Verschiebung des Nadelschutzes in Richtung der Nadel zu bilden.

4. Vorrichtung nach einem der vorhergehenden Ansprüche, dadurch gekennzeichnet, daß die Führungsfläche in einem auf die Außenfläche des Nadel-Trägerteils (2) aufgeschobenen Gleitelement (7) ausgebildet ist.

5. Vorrichtung nach einem der Ansprüche 2 bis 4, gekennzeichnet durch ein zwischen dem Nadelschutz (8) und dem Trägerteil (2, 7) der Nadel in dem mit großem Durchmesser versehenen Abschnitt (9) dieses Nadelschutzes gekoppeltes Organ (20) zur elastischen Rückstellung des Nadelschutzes in seine die Nadel vollständig verhüllende Position.

6. Vorrichtung nach einem der Ansprüche 2 bis 5, dadurch gekennzeichnet, daß der mit großem Durchmesser versehene Bereich des Nadelschutzes ein Element (21) zum Halten der Nadel (6) in der Mitte des Nadelschutzes umfaßt.
